# EUROPEAN PATENT SPECIFICATION

(11) **EP 0 319 071 B1**
(45) Date of publication and mention of the grant of the patent: **24.02.1993**
(21) Application number: 88202612.3
(22) Date of filing: 22.11.1988
(51) Int. Cl.: A61F 2/48, A61F 5/451

(54) **Stomal stop**
Stomaverschluss
Bouchon pour stoma

(30) Priority: 23.11.1987 NL 8702802; 09.03.1988 NL 8800585
(43) Date of publication of application: 07.06.1989
(73) Proprietor: Optical Disc Manufacturing Equipment B.V., 5611 CA Eindhoven (NL)
(72) Inventor: Kok, Cornelis Jacobus Maria, (NL)
(74) Representative: Hooiveld, Arjen Jan Winfried

(56) References cited:
- WO-A-87/03192
- DE-A- 2 447 996
- US-A- 1 240 125
- US-A- 2 544 201
- US-A- 2 915 065
- US-A- 3 548 828
- US-A- 4 067 335
- US-A- 4 445 899

## Description

The invention relates to a stomal stop provided with a support that is substantially circular in cross-section and whose oppositely situated ends are open, which support carries a bag open at one end.

Such a stomal stop is known from US patent No. 4,067,335. this known stomal stop comprises an annular support which is inserted into, for example, the anal canal of a patient. A folded or rolled-up bag closed at one end communicates with the support for the reception of faeces and/or urine. The excrements' passage into the bag causes the latter to be unrolled or unfolded. The part of the bag which contains the faeces and/or urine can be sealed and released, so that the remaining part of the bag still attached to the support is available for the reception of further waste matter from the patient's body.

One drawback of the known stomal stop is that it is of a rather complicated design and therefore expensive. This is a grave disadvantage, considering that a user of stomal stops requires at least on stomal stop a day. A further drawback of the known stomal stop is that it does not afford a proper sealing of the patient's anus. In addition, it has been found in actual practice that the bag of the known stomal stop is liable to be detached from its holding ring. And finally, the lack of compactness renders the known stomal stop rather unwieldy in use.

It is an object of the present invention to provide a simple, compact stomal stop which achieves a proper sealing of, for example the user's anus and in which the bag cannot get detached unintentionally from the support.

To this end, a stomal stop of the type mentioned in the introductory paragraph is according to the invention characterized in that the support surrounds the bag at least in part and that the support comprises means of attachment for securing the stomal stop in position in a skin cavity of the stomal stop user, which means of attachment contain a material which may expand through the action of heat, fluid and/or pressure, which material has been disposed at least partly round the support and/or is at least partly identical with the material from which the support has been made. Because of this design, the bag is not liable to be unintentionally detached or torn or otherwise disabled. This arrangement ensures a firm positioning of the stomal stop in, for example, a patient's natural or artificial anus.

One embodiment of a stomal stop according to the invention is characterized in that the means of attachment comprise a fluid-absorbing material which has been disposed at least partly round the support and which may expand through absorption.

A further embodiment of a stomal stop according to the invention is characterized in that before use of the stomal stop the support is at least partly enveloped in a sleeve and that the means of attachment contain a material which may expand upon removal of the sleeve, which material has been disposed at least partly round the support and/or is at least partly identical with the material from which the support has been made, whilst the said sleeve envelops the expandable material before the stomal stop is put into use. The expandable material may, for instance, be made of a polymeric substance.

A further embodiment of a stomal stop according to the invention is characterized in that the means of attachment comprise a helical spring disposed in the wall of the support, or alternatively either on the inner or on the outer wall of the support, which helical spring is tensioned before and slack after the insertion of the stomal stop into a body cavity of a stomal stop user, whilst the diameter of the helical spring is smaller when tensioned than when slack. If such a helical spring is designed so as to be hollow, gas can be passed through the cavity within the spring.

A further embodiment of a stomal stop according to the invention is characterized in that the support is surrounded at least partly by spongelike material having closed or open cells. If the spongy material contains open cells, gas can ge passed through. However, spongy material containing closed cells can readily absorb fluid.

Yet another embodiment of a stomal stop according to the invention is characterized in that the support has a conical shape and that the opening of the bag is provided with a locking ring having a larger diameter than the diameter of that end of the support which faces away from the skin cavity of a patient during use of the stomal stop, whereby during use of the stomal stop the locking ring prevents the bag from leaving the support through the said end.

The invention will now be elucidated with reference to the accompanying drawings, in which
figures 1,2, 3 and 4 represent embodiments of the stomal stop according to the invention; and
figure 5 depicts a strip of adhesive material with the aid of which the stomal stops of figures 1,2, and 3 can be stuck onto the skin of a patient with a stoma.

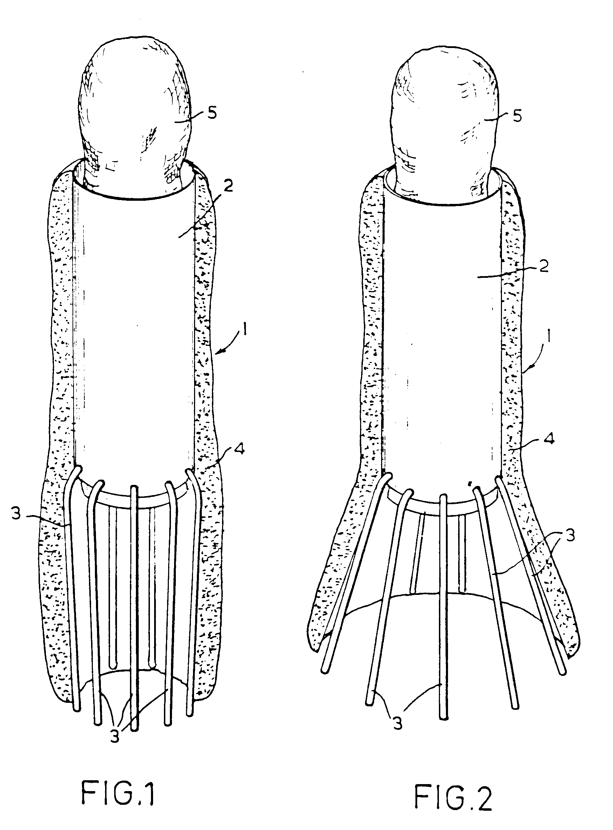

Figure 1 shows stomal stop 1 which is formed by a cylindrical support 2. At one of its ends this support 2 carries a number of resilient reeds 3. The support 2 may have been made, for example, from a flexible material. To enhance the flexibility still further, if required, the casing of the support 2 may be provided with longitudinal indentations (not shown). The support 2 is enveloped in a fluid-absorbing material 4, for example cottonwool. A bag 5 serving for the reception of faeces and/or urine is accommodated within the support 2. To permit insertion of the stomal stop 1 into the anus of a stomal stop user, the reeds 3 may be radially deflected inwards. The stomal stop 1 may, for instance, be used for an artificial, whether or not temporary, opening.

Figure 2 depicts a stomal stop similar to that of figure 1. Like parts are indicated by like reference numerals. Here the reeds 3 are radially deflected outwards. This is the position which the reeds occupy, whether or not automatically, when once the stomal stop 1 has been inserted into, for example, an anus.

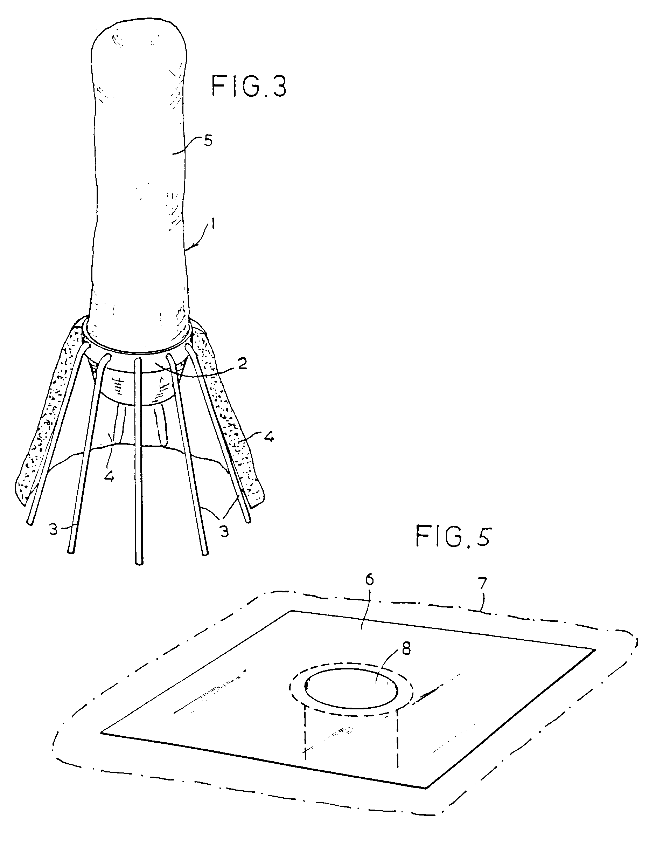

Figure 3 represents a stomal stop 1 in which the support 2 has the shape of a ring. It again comprises reeds 3, fluid-absorbing material 4 and a bag 5. In the stomal stops 1 of figures 1-4 the support 2 may, if required, include an air filter. Such an air filter may take the form of capillary ducts.

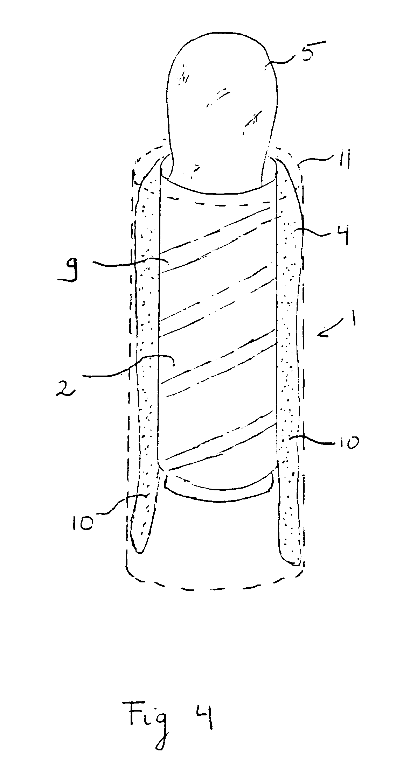

Figure 4 shows a cylindrical support 2 on whose outer wall a hollow helical spring 9 has been disposed. The support 2 thus provided with a helical spring 9 is enveloped in a cottonwool-like material 10 which may expand under the action of heat, for instance. The whole is surrounded by a cylindrical sleeve 11 which subjects the whole to a certain pre-compression. As soon as the stomal stop has been inserted into a skin cavity of a stomal stop user, the sleeve 11 is removed. As a result, the helical spring 9 will become slack - and thereby increase in diameter - which leads to a firm positioning of the stomal stop in the skin cavity. Under the influence of body heat of the stomal stop user, the cottonwool-like material 10 expands whereby the appliance is secured in position even better. Body gases of the stomal stop user can escape to atmosphere through the cavity of the helical spring 9. It will be obvious that the cottonwool-like material 10 and the helical spring 9 may be used either together or separately.

Figure 5 depicts a strip of adhesive material 6 which has been stuck onto the skin 7 of stomal stop user. The strip 6 has an opening 8 through which a stomal stop according to any one of figures 1-4 is to be inserted. A body-friendly skin spray may be applied in conjunction with the strip of adhesive material. Such a skin spray, which may contain urea derivatives, for example, is to be applied to the inner wall of a skin cavity of the stomal stop user so as to ensure that the inner wall is permeable to air but not to a fluids.

It is to be noted that the bag 5 is normally accommodated within the support 2 before use of the stomal stops represented in figures 1-4. When the stomal stops are in use, the bag 5 will gradually be filled and in consequence thereof emerge partly from the support with its closed end. If required, a locking ring is provided to prevent the bag 5 from emerging prematurely.

## Claims

1. A stomal stop (1) provided with a support (2) which is substantially circular in cross-section and whose oppositely situated ends are open, which support (2) carries a bag (5) open at one end, characterized in that the support (2) surrounds the bag (5) at least in part and that the support (2) comprises means of attachment (4,10) for securing the stomal stop (1) in position in a skin cavity of the stomal stop user, which means of attachment (4,10) contain a material which may expand through the action of heat, fluid and/or pressure, which material has been disposed at least partly round the support (2) and/or is at least partly identical with the material from which the support (2) has been made.

2. A stomal stop (1) according to claim 1, characterized in that the means of attachment (4,10) comprise a fluid-absorbing material which has been disposed at least partly round the support (2) and which may expand through absorption.

3. A stomal stop (1) according to any one of the claims 1-2, characterized in that before use of the stomal stop the support (2) is enveloped at least partly in a sleeve (11) and that the means of attachment (4,10) contain a material which may expand upon removal of the sleeve (11), which material has been disposed at least partly round the support (2) and/or is at least partly identical with the material from which the support (2) has been made, whilst the said sleeve (11) envelops the expandable material before the stomal stop (1) is put into use.

4. A stomal stop (1) according to any one of the preceding claims, characterized in that the means of attachment (4,10) comprise a helical spring (9) disposed in the wall of the support (2), which helical spring (9) is tensioned before and slack after the insertion of the stomal stop (1) into a body cavity of a stomal stop user, whilst the diameter of the helical spring (9) is smaller when tensioned than when slack.

5. A stomal stop (1) according to claim 4, characterized in that the means of attachment (4,10) comprise a helical spring (9) disposed on the inner or the outer wall of the support (2), which helical spring (9) is tensioned before and slack after the insertion of the stomal stop (1) into a body cavity of a stomal stop user, whilst the diameter of the helical spring (9) is smaller when tensioned than when slack.

6. A stomal stop (1) according to claim 4 or 5, characterized in that the helical spring (9) is hollow.

7. A stomal stop (1) according to any one of the preceding claims, characterized in that the support (2) is surrounded at least partly by spongelike material having open or closed cells.

8. A stomal stop (1) according to any one of the preceding claims, characterized in that the support (2) has a conical shape and that the opening of the bag (5) is provided with a locking ring having a larger diameter than the diameter of that end of the support (2) which faces away from the skin cavity of a patient using the stomal stop (1), whereby during use of the stomal stop (1) the locking ring prevents the bag (5) from leaving the support (2) through the said end.

9. A stomal stop (1) according to any one of the preceding claims, characterized in that the support (2) has been made from a flexible material.

10. A stomal stop (1) according to any one of the preceding claims, characterized in that the support (2) has a substantially cylindrical shape.

11. A stomal stop (1) according to any one of the preceding claims 1-9, characterized in that the support (2) has substantially the shape of a ring.

12. A stomal stop (1) according to claim 10 or 11, characterized in that at least one indentation has been made in at least part of the surface of the support (2).

13. A stomal stop (1) according to any one of the preceding claims, characterized in that the support (2) includes an air filter.

14. A stomal stop (1) according to any one of the preceding claims, characterized in that in the operating condition of the stomal stop (1) the support (2) has been passed through a strip (6) of adhesive material by means of which the support has been stuck onto the skin (7) of a stomal stop user.

15. A stomal stop (1) according to any one of the preceding claims, characterized in that, in order to facilitate the insertion of the stomal stop (1) into a skin cavity of a stomal stop user, a film has been applied round about the stomal stop (1), which film can be removed after the insertion.

16. A stomal stop (1) according to any one of the preceding claims, characterized in that an inflatable pouch has been applied round about the support (2) for adaptation of the stomal stop (1) to the size of a skin cavity of a stomal stop user.

## Patentansprüche

1. Stomaler Verschluß (1), der mit einem Träger (2) versehen ist, welcher im Querschnitt im wesentlichen kreisförmig ist und dessen gegenüberliegenden Enden offen sind, wobei der Träger (2) einen an einem Ende offenen Beutel (5) trägt, dadurch gekennzeichnet, daß der Träger (2) den Beutel (5) wenigstens teilweise umgibt und daß der Träger (2) Anbringungsmittel (10) zur Befestigung des stomalen Verschlußes (1) an Ort und Stelle in einer Hauttasche des Verwenders des stomalen Verschlußes aufweist, das ein Material beinhaltet, welches sich durch die Wirkung von Wärme, einer Flüssigkeit und/oder Druck ausdehnen kann und wenigstens teilweise um den Träger (2) angeordnet ist und/oder wenigstens teilweise mit dem Material, aus dem der Träger (2) gefertigt ist, identisch ist.

2. Stomaler Verschluß (1) nach Anspruch 1, dadurch gekennzeichnet, daß die Mittel zur Anbringung (4, 10) ein eine Flüssigkeit absorbierendes Material aufweisen, das wenigstens teilweise um den Träger (2) angeordnet ist und das sich durch Absoption ausdehnen kann.

3. Stomaler Verschluß nach einem der Ansprüche 1 - 2, dadurch gekennzeichnet, daß der Träger (2) vor der Verwendung des stomalen Verschlußes wenigstens teilweise von einer Manschette (11) umgeben ist und daß das Anbringungsmittel (4, 10) ein Material beinhaltet, das sich bei Entfernung der Manschette (11) ausdehen kann, wobei das Material wenigstens teilweise um den Träger (2) angeordnet ist und/oder wenigstens teilweise mit dem Material, aus dem der Träger (2) gebildet ist, identisch ist, wobei die Manschette (11) das ausdehnungsfähige Material umgibt, bevor der stomale Verschluß (1) in Verwendung kommt.

4. Stomaler Verschluß (1) nach einem der vorangehenden Ansprüche, dadurch gekennzeichnet, daß das Anbringungsmittel (4, 10) eine Schraubenfeder (9) aufweist, die in der Wandung des Trägers (2) angeordnet ist, wobei die Schraubenfeder (9) vor dem Einsetzen des stomalen Verschlußes (1) in einer Körperhöhlung des Verwenders des stomalen Verschlußes gespannt und danach entspannt wird, wobei der Durchmesser der Schraubenfeder (9) bei Spannung kleiner ist als bei Entspannung.

5. Stomaler Verschluß (1) nach Anspruch 4, dadurch gekennzeichnet, daß das Anbringungsmittel (4, 10) eine Schraubenfeder (9) aufweist, die an der inneren oder der äußeren Wandung des Trägers (2) angeordnet ist, wobei die Schraubenfeder vor dem Einsetzen des stomalen Verschlußes (1) in eine Körperhöhlung des Verwenders des stomalen Verschlußes gespannt und danach entspannt wird, wobei der Durchmesser der Schraubenfeder (9) bei Spannung kleiner ist als bei Entspannung.

6. Stomaler Verschluß (1) nach Anspruch 4 oder 5, dadurch gekennzeichnet, daß die Schraubenfeder (9) hohl ist.

7. Stomaler Verschluß (1) nach einem der vorangehenden Ansprüche, dadurch gekennzeichnet, daß der Träger (2) wenigstens teilweise durch ein schaumartiges Material mit offenen oder geschlossenen Zellen umgeben ist.

8. Stomaler Verschluß (1) nach einem der vorangehenden Ansprüche, dadurch gekennzeichnet, daß der Träger (2) eine konische Form hat und daß die Öffnung des Beutels (5) mit einem Verschlußring versehen ist, dessen Durchmesser größer als der des Endes des Trägers (2) ist, daß von der Hauttasche des Patienten, der den stomalen Verschluß (1) trägt, wegweist, wobei der Verschlußring während der Verwendung des stomalen Verschlußes (1) den Beutel (5) daran hindert, sich von dem Träger (2) durch das Ende zu lösen.

9. Stomaler Verschluß (1) nach einem der vorangehenden Ansprüche, dadurch gekennzeichnet, daß der Träger (2) aus einem flexiblen Material gefertigt ist.

10. Stomaler Verschluß (1) nach einem der vorangehenden Ansprüche, dadurch gekennzeichnet, daß der Träger (2) eine im wesentlichen zylindrische Form hat.

11. Stomaler Verschluß (1) nach einem der vorangehenden Ansprüche 1 - 9, dadurch gekennzeichnet, daß der Träger (2) im wesentlichen ringförmig ist.

12. Stomaler Verschluß (1) nach einem der Ansprüche 10 oder 11, dadurch gekennzeichnet, daß wenigstens ein Einschnitt in wenigstens einen Teil der Oberfläche des Trägers (2) eingebracht ist.

13. Stomaler Verschluß (1) nach einem der vorangehenden Ansprüche, dadurch gekennzeichnet, daß der Träger (2) ein Luftfilter aufweist.

14. Stomaler Verschluß (1) nach einem der vorangehenden Ansrüche, dadurch gekennzeichnet, daß bei einem Betriebszustand des stomalen Verschlußes (1) der Träger (2) durch einen Streifen (6) aus einem Klebematerial geführt ist, mittels dessen der Träger auf die Haut (7) des Verwenders eines stomalen Verschlußes aufgeklebt ist.

15. Stomaler Verschluß (1) nach einem der vorangehenden Ansprüche, dadurch gekennzeichnet, daß zur Ermöglichung des Einsetzens des stomalen Verschlußes (1) in eine Hautöffnung des Verwenders des stomalen Verschlußes ein Film um den stomalen Verschluß (1) aufgebracht ist, der nach dem Einsetzen entfernt werden kann.

16. Stomaler Verschluß (1) nach einem der vorangehenden Ansprüche, dadurch gekennzeichnet, daß ein aufblasbarer Beutel um den Träger (2) aufgebracht ist. Zur Anpassung des stomalen Verschlusses (1) an die Größe der Hauttasche des Verwenders eines stomalen Verschlusses.

## Revendications

1. Bouchon pour stoma (1) comportant un support (2) qui a une section sensiblement circulaire et dont les extrémités opposées sont ouvertes, lequel support (2) porte un sac (5) ouvert à une de ces extémités caractérisé en ce que le support (2) entoure le sac (5) au moins en partie et en ce que le support (2) comporte des moyens de fixation (4,10) pour maintenir le bouchon pour stoma (1) en position dans une cavité de peau de l'utilisateur du bouchon pour stoma, lesquels moyens de fixation (4,10) contiennent un matériau qui peut se dilater sous l'action de la chaleur, d'un fluide et/ou par la pression, lequel matériau a été disposé au moins en partie autour du support (2) et/ou se trouve au moins en partie identique au matériau à partir duquel le support (2) a été réalisé.

2. Bouchon pour stoma (1) selon la revendication 1 caractérisé en ce que les moyens de fixation (4,10) comportent un matériau qui absorbe du fluide lequel a été disposé au moins en partie autour du support (2) et peut se dilater par absorption.

3. Bouchon pour stoma (1) selon l'une quelconque des revendications 1 ou 2, caractérisé en ce qu'avant utilisation du bouchon pour stoma, le support (2) est entouré au moins partiellement par un fourreau (11) et en ce que les moyens de fixation (4,10) contiennent un matériau qui peut se dilater lors de l'extraction du fourreau (11), lequel matériau a été disposé au moins en partie autour du support (2) et/ou qui est au moins partiellement identique au matériau à partir duquel le support (2) a été réalisé, ledit fourreau (11) entourant le matériau dilatable avant l'utilisation du bouchon pour stoma (1).

4. Bouchon pour stoma (1) selon l'une quelconque des revendications précédentes caractérisé en ce que les moyens de fixation (4,10) comportent un ressort hélicoïdal (9) disposé dans la paroi du support (2), lequel ressort hélicoïdal (9) est tendu avant et relâché après l'insertion du bouchon pour stoma (1) dans une cavité du corps d'un utilisateur du bouchon pour stoma, le diamètre du ressort hélicoïdal (9) étant plus petit lorsqu'il est tendu que lorsqu'il est relâché.

5. Bouchon pour stoma (1) selon la revendication 4 caractérisé en ce que les moyens de fixation (4,10) comportent un ressort hélicoïdal (9) disposé sur la paroi intérieure ou extérieure du support (2), lequel ressort hélicoïdal (9) est tendu avant et relâché après l'insertion du bouchon pour stoma (1) dans une cavité du corps du porteur de bouchon pour stoma, le diamètre du ressort helicoïdal (9) étant plus petit quand il est tendu que quand il est relâché.

6. Bouchon pour stoma (1) selon la revendication 4 ou la revendication 5 caractérisé en ce que le ressort hélicoïdal (9) est creux.

7. Bouchon pour stoma (1) selon l'une quelconque des revendications précédentes caractérisé en ce que le support (2) est entouré au moins partiellement par un matériau spongieux comportant des cellules ouvertes ou fermées.

8. Bouchon pour stoma (1) selon l'une quelconque des revendications précédentes caractérisé en ce que le support (2) est de forme conique et en ce que l'ouverture du sac (5) est prévue avec une bague de verrouillage ayant un diamètre plus grand que le diamètre de l'extrémité du support (2) qui se trouve éloignée de la cavité de peau d'un patient utilisant le bouchon pour stoma (1), de sorte qu'au cours de l'utilisation du bouchon pour stoma (1) la bague de verrouillage empêche le sac (5) de quitter le support (2) par ladite extrémité.

9. Bouchon pour stoma (1) selon l'une quelconque des revendications précédentes caractérisé en ce que le support (2) est réalisé en un matériau élastique.

10. Bouchon pour stoma (1) selon l'une quelconque des revendications précédentes caractérisé en ce que le support (2) est de forme sensiblement cylindrique.

11. Bouchon pour stoma (1) selon l'une quelconque des revendications précédentes 1-9 caractérisé en ce que le support (2) a sensiblement la forme d'une bague.

12. Bouchon pour stoma (1) selon la revendication 10 ou la revendication 11 caractérisé en ce qu'une entaille a été réalisée dans au moins une partie de la surface du support (2).

13. Bouchon pour stoma (1) selon l'une quelconque des revendications précédentes caractérisé en ce que le support (2) comporte un filtre à air.

14. Bouchon pour stoma (1) selon l'une quelconque des revendications précédentes caractérisée en ce que lorsqu'il est en condition de fonctionnement, le support (2) passe à travers une bande (6) de matériau adhésif au moyen duquel le support se trouve collé sur la peau de l'utilisateur d'un bouchon pour stoma.

15. Bouchon pour stoma (1) selon l'une quelconque des revendications précédentes caractérisé en ce que, pour faciliter l'insertion du bouchon pour stoma (1) dans une cavité de peau d'un utilisateur de bouchon pour stoma, un film a été appliqué autour du bouchon de stoma (1), lequel film peut être éliminé après l'insertion.

16. Bouchon pour stoma (1) selon l'une quelconque des revendications précédentes caractérisé en ce qu'une poche gonflable a été appliquée autour du support (2) en vue de l'adaptation du bouchon pour stoma (1) à la dimension de la cavité de peau d'un utilisateur de bouchon pour stoma.
